# EUROPEAN PATENT APPLICATION

(11) **EP 1 884 256 A1**
(43) Date of publication of application: **06.02.2008**
(21) Application number: 07113290.6
(22) Date of filing: 27.07.2007
(51) Int. Cl.: A61M 16/00

(54) **Pressure targeted ventilator using an oscillating pump**

(30) Priority: 02.08.2006 US 461792
(71) Applicant: General Electric Company, Schenectady, NY 12345 (US)
(72) Inventor: Kollmeyer, Phillip J., Fond du Lac, WI 54935 (US); Tham, Robert Q., Middleton, WI 53562 (US); Rick, Norman, Mount Horeb, WI 53572 (US)
(74) Representative: Illingworth-Law, William Illingworth

(57) **Abstract**

A respiratory support system for providing respiratory support to a patient (10) comprising an oscillating pump (12). The oscillating pump (12) pressurizes ambient air (14) to a pressure suitable for delivery to the patient (10) for respiratory support. The system further comprises a sensor (20) and a microprocessor (28) to control the oscillating pump (12) in response to patient breath attempts.

## Description

### FIELD OF THE INVENTION

The present invention is directed to the field of systems for providing a patient with respiratory assistance including anesthesia delivery and ventilation support. Specifically, the present invention aims to increase the portability while decreasing the cost to operate these systems.

### BACKGROUND OF THE INVENTION

Patients that have respiratory difficulties often must be placed on a mechanical ventilator. These difficulties may by pathological in nature or may be due to the fact that the patient is too weak or sedated to independently perform respiration functions. Often, the patient may be spontaneously attempting to breathe but not able to complete a full respiratory cycle. In these cases, mechanically assisted ventilation is provided. In some mechanically assisted ventilation platforms, a combination of pressure and/or flow sensors detect a patient's breath attempt. This detection triggers the delivery of a mechanical breath. This breath is provided by the delivery of medical gases under a pressure that is sufficient to overcome the resistance of the patient's airway to fill the lungs. When the pressure of the medical gas is reduced, the natural compliance of the patient's chest wall forces the delivered breath out of the patient in expiratory phase.

A variety of systems are available to produce the pressure needed to deliver a breath of medical gas. The pressure may be supplied from pressurized medical gas supply tanks. These supply tanks may be large, institutionally installed tanks with individual supply lines running to specific rooms and/or locations within a treatment facility. While this system provides an economy of scale on supplying medical gases, the infrastructure required greatly limits the locations that ventilation can be performed to the areas that have been supplied with supply lines. Alternatively, smaller and thus more portable medical gas supply tanks may be used by a ventilatory system. But these tanks are more expensive and, while mobile, are still cumbersome to transport. As with any ventilation system that uses the pressure from pressurized medical gas supply tanks, control valves in the ventilator provide a resistance to the pressurized gas, thereby controlling the pressure of the gas supplied to the patient.

The medical gases supplied to the patient may comprise air or supplemental gases such as oxygen, helium, nitric oxide, anesthetic agent or a drug aerosol. However, air is the greatest proportion of the medical gas that is supplied to the patient. Air is referred to as the drive gas for the ventilator system and any other medical gases are referred to as supplemental gases to the air. Because of the abundance of the surrounding air, an alternative solution for creating the necessary ventilation pressure is to use a motor that can take the ambient air from outside the ventilator and pressurize it. The two most common types of systems that are used for this process are the piston or bellows system and the rotary or fan system. Piston or bellows type pumps are usually utilized in anesthesia delivery machines. These pumps are typically large in size as they require an air bag or bellows. While this reduces the weight of carrying pressurized supply tanks, these systems can still be cumbersome to move.

Rotary or fan style pumps are more compact than piston pumps, but rotary pumps have their disadvantages as well. Specifically, rotary pumps have a slow response time, therefore taking a long time to cycle from a low flow rate to a high flow rate. This is a big disadvantage as proper patient ventilation requires detailed control of the pressure created by the ventilator to enable pressure changes to correspond with the patient's breathing cycle. This disadvantage can be overcome by running the rotary pumps at a continuous high flow rate and by adding additional control valves and circuitry to change the pressure of the medical gas that is delivered to the patient, similar to that used with the pressurized gas supply tanks. While this provides the response time necessary for proper patient ventilation, this system consumes a large amount of electricity as the rotary pump must be continuously run at its top speed and the additional control valves and circuitry must be powered.

Therefore, a patient respiratory support system that can provide sufficient medical gas pressure to ensure proper patient ventilation, provide a fast response time to continually adjust the pressure delivered in conjunction with the patient's respiratory cycle, and provide low power consumption is desired. A patient respiratory support system that uses a pump that combines these qualities would greatly increase the portability of patient respiratory support systems, thus allowing greater flexibility in locations where patients may receive respiratory support.

### SUMMARY OF THE INVENTION

The present invention provides a patient respiratory support system that is portable, has a fast response time, and conserves electrical power. The patient respiratory support system of the present invention utilizes a linear oscillating pump to pressurize the medical gas to be delivered to the patient. Linear pumps are currently used in many applications where low noise, low pressure, high flow rates, and dependable operation are necessary, however until now have not been used in the field of patient respiratory support. Linear oscillating pumps are most utilized in water aeration applications where variable flow operation is not needed.

The linear oscillating pump as used in the present invention is controlled by a motor controller that controls the magnitude of the pressure generated by the pump. When used to generate pressures in the range of those typically supplied to the patient airway (0-120 cm H₂O, typical), this linear pump configuration performs more favorably than either the rotary or the piston pump in this application. The linear oscillating pump provides a fast time response to commanded flow changes while providing a very low power consumption because it produces flow on demand.

### BRIEF DESCRIPTION OF THE DRAWINGS

The drawings illustrate embodiments, provided by way of example, including the best mode contemplated of carrying out the invention, in which:
Figure 1 is a schematic diagram of the present invention as it is used to deliver ventilatory support to a patient;
Figure 2 is an overhead view of the general physical structure of the linear oscillating pump of the present invention;
Figure 3 is a side view of the motor core and windings cut along line 3-3.
Figure 4 is a schematic diagram of the electrical signals used to drive the linear oscillating pump of the present invention;
Figure 5A is a graph depicting the output pressure of the linear pump before receiving pressure damping;
Figure 5B is a graph of the output pressure of the linear oscillating pump after receiving pressure damping;
Figure 6 is a schematic diagram of a ventilator system of the present invention utilizing post-pump supplemental oxygen mixing; and
Figure 7 is a schematic diagram of a ventilator system of the present invention utilizing pre-pump supplemental oxygen mixing.

### DETAILED DESCRIPTION OF THE INVENTION

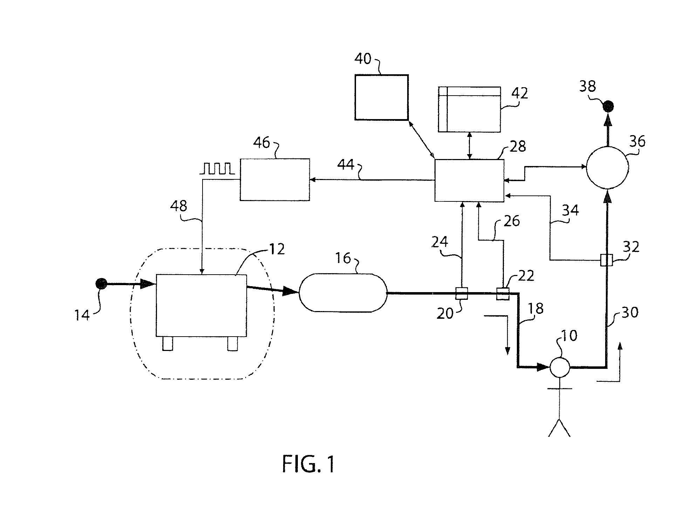

Figure 1 is a schematic diagram of a ventilator system of the present invention for providing respiratory support to a patient 10. The linear oscillating pump 12 takes outside air and pumps it into the system at a predetermined pressure. Once the pressurized air leaves the linear oscillating pump 12, it receives mechanical damping 16 to reduce the effects of the oscillation in the air flow. The pressurized air is then delivered to the patient 10 via an inspiratory conduit 18. A variety of sensors may be disposed within the inspiratory conduit 18 to monitor the respiratory support that is being provided to the patient 10. These sensors may include but should not be read as limited to pressure sensors 20 and flow sensors 22. These sensors send their respective pressure signal 24 and flow signal 26 to a microprocessor 28. Alternatively, the sensors may be disposed within the patient breathing connection (not pictured) for the detection of pressure or flow changes due to patient breath attempts. The patient connection may comprise a mask, a breathing helmet, an endotrachial tube, a nasal cannula or any other suitable patient connection for delivering medical gas to a patient.

As the patient 10 enters the expiratory phase of the respiratory cycle, the expired breathing gases are channeled to an expiratory conduit 30. Additional sensors may be disposed along the expiratory conduit 30, such as the flow sensor 32 depicted, but should not be read as limiting as other sensors, or none at all may be included in the expiratory conduit 30 as embodiments of the present invention. The flow sensor 32 will send a signal 34 to the microprocessor 28 representative of the flow of gas in the expiratory conduit 30. Alternatively, sensor 32 may be a pressure transducer, measuring the pressure within the expiratory conduit 30. At the end of the expiratory conduit 30 is an expiratory valve 36 which upon receiving a control signal from the microprocessor 28 releases the expired gases to the outside 38.

As the microprocessor 28 receives various signals (24, 26, 34) from the system, this information may be sent to a user interface 40 where it may be viewed by a clinician. The clinician may also use the user interface 40 to enter desired parameters and/ or controls for the respiratory support that is being provided to the patient 10. The microprocessor 28 will use the clinician-defined controls as well as the received signals (24, 26, 34) as interpreted using stored reference calibration tables 42 to provide a control signal 44 to the motor controller 46. This control signal 44 will vary dependent with the stage the patient is in the respiratory cycle. If the patient is making a breath attempt, the control signal 44 will indicate that more pressure is needed. If the patient is exhaling, the control signal 44 will indicate that less pressure is needed to conserve energy.

The control signal 44 may control the motor controller 46 using pulse width modulation. Pulse width modulation (PWM) is used by the motor controller 46 to emulate an AC power signal 48 of variable fundamental frequency, variable voltage or current amplitude, and/or variable waveform. The power signal emulated could be a sine wave or any combination of sine waves of variable frequency and amplitude. A linear amplifier may also be used to generate this power signal 48. The power signal 48 drives the pump. For the present description, PWM generating a constant frequency, variable voltage amplitude, square waveform power signal 48 will be used, but is not intended to be limiting. The AC power signal 48 is provided to the pump 12. As the input voltage varies, it in effect varies the amplitude of the pump oscillation and correspondingly varies the flow output. This is an effective design for varying input voltage due to the large inductance of the pump that prevents significant pulsing in the motor current.

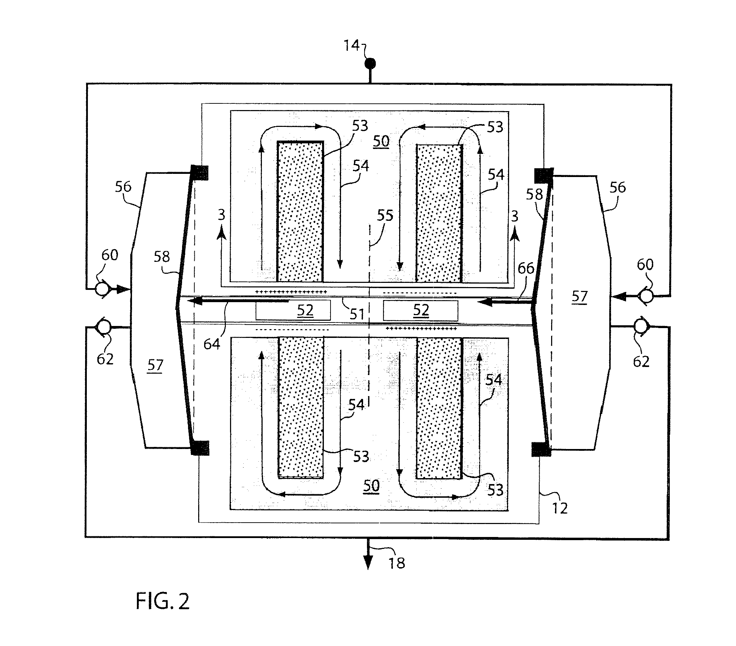

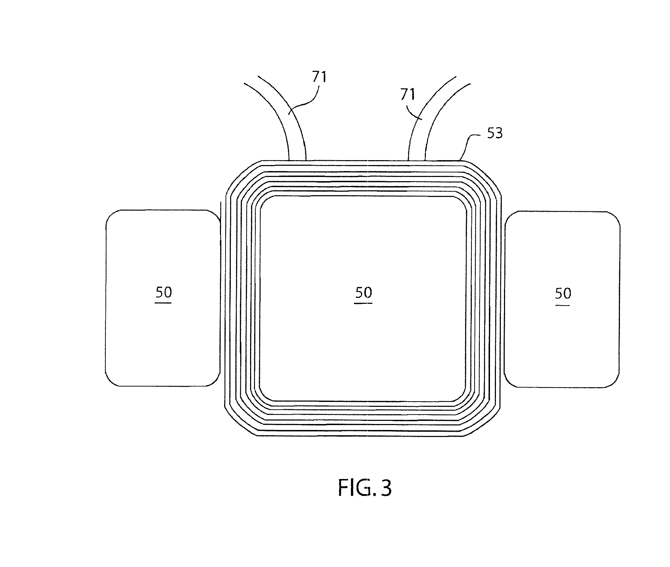

Figure 2 is an overhead view of the general physical structure of an embodiment of the linear oscillating pump 12 as depicted in Figure 1. As depicted in Figure 3, which is a cutaway view of the pump along line 3-3, the linear oscillating pump 12 comprises a stator, comprising two annular ferromagnetic laminated motor cores 50 and a plurality of motor windings 53. Referring back to Figure 2, the pump 12 also comprises a rotor 51. In a preferred embodiment, the rotor 51 is a linearly moving rotor 51, however it is understood that an oscillating pump within the scope of the present invention may produce alternative directions of rotor movement. The rotor 51 comprises two magnets 52, one disposed at either end of the rotor 51.

When an electrical current is applied to the motor windings 53 via leads 71, a magnetic field is produced within the motor cores in the exemplary direction of magnetic field lines 54. These fields push both magnets 52 in the rotor 51 in the same direction, thereby moving one end of the rotor 51 towards the outside of the linear oscillating pump 12 and the other magnet towards the center line 55 of the linear oscillating pump 12.

A pump assembly 56 is disposed on either side of the linear oscillating pump 12 in coaxial relationship to both the motor cores 50 and the magnets 52. Each pump assembly 56 comprises a rubber diaphragm 58, defining a pump chamber 57, an "in" one-way valve 60, and an "out" one-way valve 62.

As the magnetic field 54 through the motor cores 50 forces one of the magnets 52 towards the outside of the linear oscillating pump 12, this electrical force 64 pushes the rubber diaphragm 58 outwards thereby forcing the air in the pump chamber 57 out through the "out" one-way valve 62 into inspiratory conduit 18. On the other side of the linear oscillating pump 12, where the magnet 52 is proximal to the centerline 55 of the pump 12, this produces a mechanical force 66 pulling the rubber diaphragm 58 towards the center of the pump which pulls outside air 14 through the "in" one-way valve 60 to be stored in the pump chamber 57.

When the electrical charges on the motor cores 50 are reversed, the magnetic fields depicted by magnetic field lines 54 reverse forcing the magnets 52 in the opposite direction. As such, the air in the full pump assembly 56 is forced out through the "out" one-way valve 62 and into the inspiratory conduit 18 while the other pump assembly 56 that had been previously emptied now begins to fill with air from outside 14 through the "in" one-way valve 60. This cycle of charges on the motor cores 50 produces the desired output of pressurized gas into the inspiratory conduit 18.

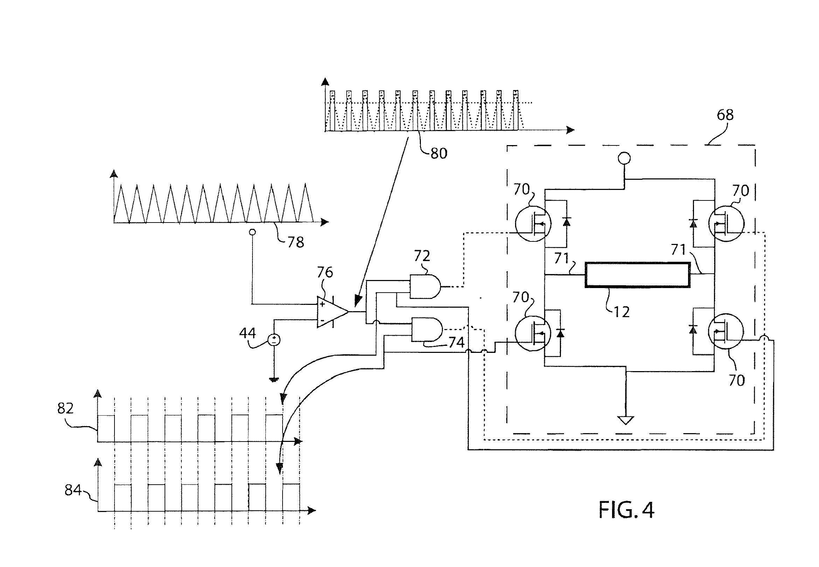

Figure 4 depicts a schematic diagram of the electrical signals that are sent via leads 71 to the motor windings 53 of the linear oscillating pump 12. As an embodiment of the present invention, the motor windings 53 are controlled using an H-bridge inverter 68. It is understood that there are a variety of other DC to AC inversion methods for motor controllers that could be used in the present invention, including digital controllers. The H-bridge inverter 68 comprises four MOSFETs 70. These MOSFETs 70 receive signals from either a first AND gate 72, a second AND gate 74, and from square wave signal 82 or 84. Both the first 72 and second 74 AND gates receive a signal from a comparator 76 which compares a triangular wave 78 with the flow control signal 44 received from the microprocessor 28. The product from the triangle wave 78 and the flow control signal 44 sent to the comparator 76 produces a comparator signal 80 that is indicative of the desired duty cycle for the operation of the linear oscillating pump. A first square wave signal 82 is provided as the second input to the first AND gate 72 and a second square wave signal 84 that is the same frequency as, but 180 degrees out of phase with, the first square wave signal 82 is provided to the second AND gate 74.

In an embodiment of the present invention, the duty cycle of these two square waves is slightly less than 50% to create a dead time between switching voltage polarity of the pump. The creation of dead time prevents shoot through, the case when two mosfets on the same side of the H-Bridge are active and the positive voltage is therefore shorted to ground. First square wave 82 and second square wave 84 operate at a frequency that is equal to that of the motor AC operating frequency. The triangular wave 78 operates at a frequency much greater than the motor operating frequency to implement PWM voltage magnitude control. In a preferred embodiment, the motor drive signal is a 24 V 60 Hz pulse width modified square wave with the effective voltage magnitude of the wave being defined by the output signal 80 of the comparator 76. However, it is understood that any periodic signal capable of producing an oscillating motion in an oscillating pump may be used in accordance with the present invention.

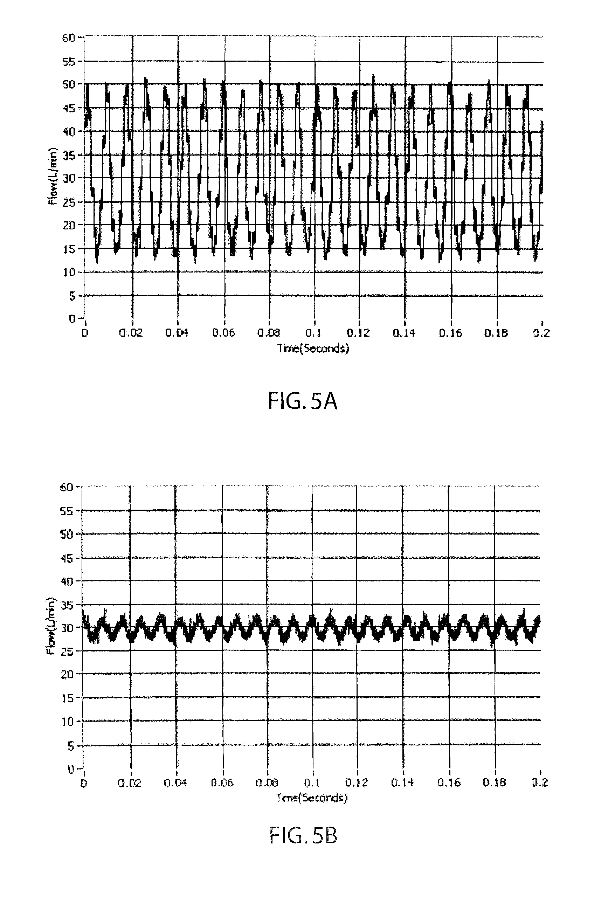

The linear motor driven diaphragm 58 displaces air at a rate dependent on rotor position, thus causing the flow output to oscillate over each individual stroke of the rotor. This flow oscillation resembles a sine wave of a frequency that is twice the motor's electrical frequency. Such flow rate oscillation is depicted in Figure 5A. Therefore, before the inspiratory flow is delivered to the patient, mechanical damping is applied to the pressurized gas to remove much of the oscillatory characteristic, as depicted in Figure 5B, making the delivered gas flow more suitable for delivery to the patient.

In embodiments of the present invention, the medical gas delivered to the patient is not limited to ambient air. The respiratory support system of the present invention may be implemented in conjunction with the delivery of an anesthetic agent. Patients receiving an anesthetic agent also often receive mechanical respiratory support as may be provided by the system of the present invention. Alternatively, in an embodiment of the present invention, the respiratory support system may be used in conjunction with the delivery of supplemental oxygen. The supplemental oxygen mixed with the delivery of the ambient air increases the oxygen content of the medical gas that is delivered to the patient and as such increases the efficiency of the gas exchange within the patient's lungs.

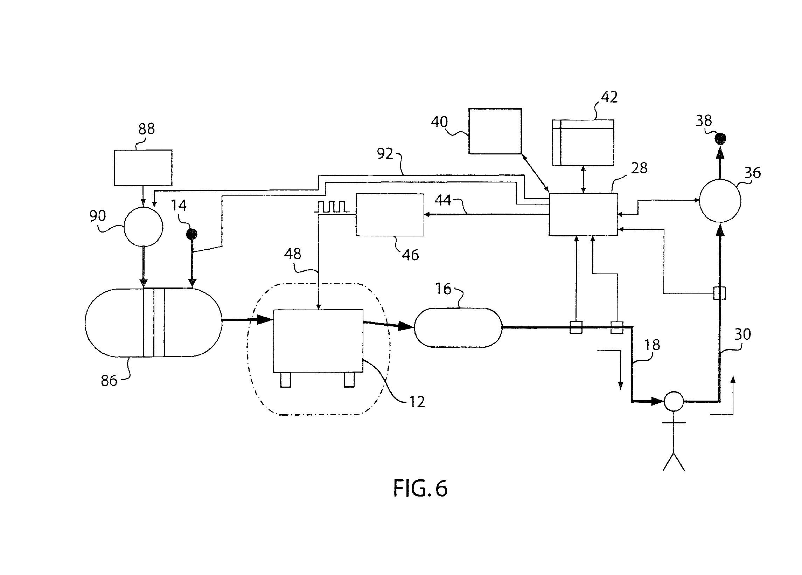

Two possible configurations for a ventilator system of the present invention using supplemental oxygen are configurations where the supplemental oxygen is added before the linear pump and configurations where the supplemental oxygen is added after the linear pump. Figure 6 depicts the configuration of a ventilator system utilizing a pre-pump supplemental oxygen source. A gas mixing reservoir 86 is used to combine outside air 14 with supplemental oxygen. The supplemental oxygen would be provided from a tank of pressurized oxygen 88 or other oxygen source and the amount of supplemental oxygen would be controlled via a control valve 90 that receives a control signal 92 from the microprocessor 28. The amount of supplemental oxygen to be combined with the ambient air could be determined automatically by the microprocessor 28 or could be a value entered by a clinician via the user interface 40.

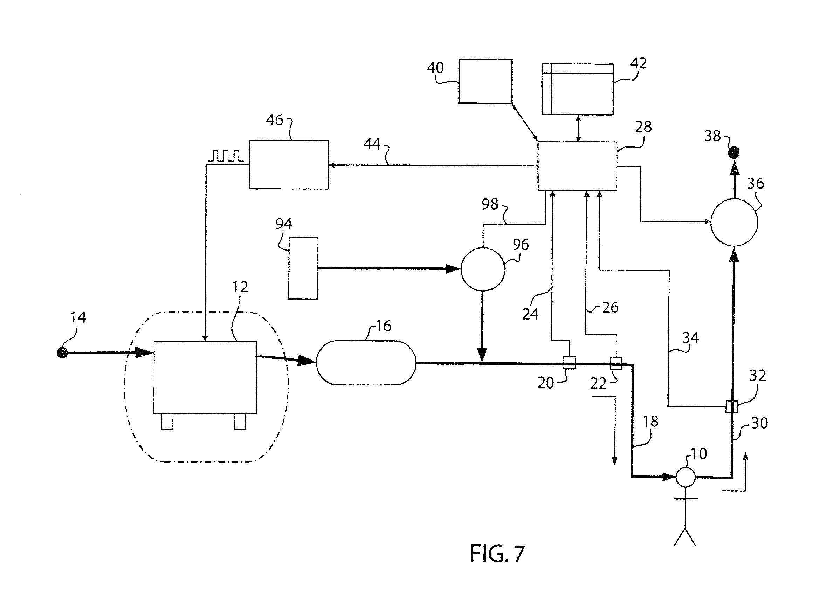

Figure 7 is a schematic diagram of a ventilator system of the present invention utilizing post-pump supplemental oxygen mixing. In this configuration, pressurized oxygen from a supply tank 94 is added to the inspiratory conduit 18 at a point in the conduit after the flow oscillation damping system 16. The flow of the pressurized oxygen is controlled by a flow valve 96 which is controlled by a signal 98 from the microprocessor 28. The addition of the supplemental oxygen at this point close to the patient 10 requires more complex algorithms in the microprocessor 28 and control by the flow valve 96 as the air to be delivered to the patient 10 has already been pressurized by the linear pump 12. However, this configuration provides the additional benefit of not having enriched air pass through the linear pump. This allows the air to enter the pump and cool the windings and core material of the pump increasing the efficiency while slightly warming the air to be delivered to the patient.

In an embodiment of the present invention, the linear oscillating pump of the present invention is not limited to the pump described above. Embodiments of the present invention may use an oscillating pump to produce non-linear motion by a rotor. This type of oscillating pump may produce rotational rotor movement or exert an alternative force on an alternative rotor such as a spring. Further embodiments of the linear oscillating pump as used in accordance with the present invention may comprise a single diaphragm.

The present invention presents the advantage of having very low average power consumption. The linear oscillating pump is efficient at producing low flow rates, but the power efficiency of the pump decreases as the flow rate increases. However, the majority of flow rates required to supplement an average breathing cycle are in the range of 0 to 40 liters per minute. These flow rates are well within the range where the pump efficiency is very high. As an example of the efficiency of the present invention, a laboratory test found that a respiratory support system driven by a flow diverting rotary pump consumed an average of 84 W while a similarly performing respiratory support system of the present invention utilizing a linear pump averaged only 2.9 W of power consumption over the same time period.

The electrical characteristics of the linear pump of the system of the present invention give this invention the advantage of a rapid response time to reach a designated target output pressure. During the acceleration of the linear pump, there are no large starting currents, low starting torque problems, nor are there any complex combinations of input voltage and electrical frequency necessary to start the motor as is often necessary in rotary devices. The linear pump takes approximately one full cycle of operation to accelerate to peak output and requires no special controls. As an example, at an electrical frequency of 60 Hz, the linear pump requires approximately 20 ms to accelerate to full flow output. This fast flow acceleration creates desirable response times for reaching target pressures. This facilitates delivering medical gas to the patient in conjunction with the patient entering the inspiratory phase of the respiration cycle.

The present invention also presents the additional benefit of added maintenance efficiency. Stock linear pumps have a long MTBF (mean time between failure) thereby running efficiently for a long time without need for replacement. This is directly related to the simple design of the linear pump and the absence of frictional moving parts. Therefore, the present invention has the additional benefit of requiring relatively low maintenance compared to current designs.

This written description uses examples to disclose the invention, including the best mode, and also to enable any person skilled in the art to make and use the invention. The patentable scope of the invention is defined by the claims and may include other examples that occur to those skilled in the art. Such other examples are intended to be within the scope of the claims if they have structural elements that do not differ from the literal language of the claims, or if they include equivalent structural elements of insubstantial difference from the literal language of the claims.

Various alternatives and embodiments are contemplated as being with in the scope of the following claims, particularly pointing out and distinctly claiming the subject matter regarded as the invention.

## Claims

1. A respiratory support system, for providing respiratory support to a patient (10), the system comprising:
an inlet for the introduction of medical gas to the system;
an oscillating pump (12) for pressurizing the medical gas to a target pressure;
a conduit (18) for delivering the medical gas to the patient; and
a sensor (20) disposed in the conduit;
wherein the sensor (20) monitors a parameter, the parameter being sent to a microprocessor that controls the oscillating pump (12) in response to changes in the parameter.

2. The respiratory support system of claim 1 wherein the oscillating pump (12) is a pump which oscillates one or more diaphragms (58) multiple strokes per breath delivered to the patient.

3. The respiratory support system of claim 1 wherein the medical gas is air and inlet for the introduction of medical gas to the system is connected to ambient air.

4. The respiratory support system of claim 3 further comprising the addition of a supplemental medical gas.

5. The respiratory support system of claim 4 wherein the supplemental medical gas is Oxygen.

6. The respiratory support system of claim 5 wherein the oxygen is added to the air after it has been pressurized by the oscillating pump (12).

7. The respiratory support system of any one of the preceding claims, wherein the parameter is the pressure of air within the conduit.

8. The respiratory support system of claim 1 wherein the microprocessor (28) uses a calibration table (42) to interpret the parameter to select a proper duty cycle for the pump.

9. The respiratory support system of claim 1 further comprising a user interface (40) wherein a clinician may use the user interface (40) to modify the parameter.

10. The respiratory support system of claim 1 further comprising a flow oscillation damping means (16) wherein the pressurized medical gas is prepared for delivery to the patient (10).
